# EUROPÄISCHE PATENTSCHRIFT

(11) **EP 1 405 863 B1**
(45) Veröffentlichungstag und Bekanntmachung des Hinweises auf die Patenterteilung: **13.02.2008**
(21) Anmeldenummer: 03020148.7
(22) Anmeldetag: 05.09.2003
(51) Int. Cl.: C07K 14/755, A61K 38/37, A61P 7/04

(54) **Konzentrat eines Faktor VIII:C-haltigen von-Willebrand-Faktors und das dazu gehörige Verfahren**
Concentrate of a factor VIII:C-containing von-Willebrand-factor and a process for producing it
Concentré de facteur von-Willebrand comprenant de facteur VIII:C et son procédé de production

(30) Priorität: 01.10.2002 DE 10246125
(43) Veröffentlichungstag der Anmeldung: 07.04.2004
(73) Patentinhaber: CSL Behring GmbH, 35041 Marburg (DE)
(72) Erfinder: Kumpe, Gerhardt, 35083 Wetter (DE); Juraschek, Manfred, 35096 Weimar (DE); Mayer, Natascha, 35039 Marburg (DE); Schulte, Stefan, Dr., 35043 Marburg (DE); Wormsbächer, Wilfried, 35274 Kirchhain (DE)

(56) Entgegenhaltungen:
- WO-A-98/38218
- US-A- 4 361 509
- US-A- 5 288 853
- US-B1- 6 228 613
- HEIMBURGER N ET AL.: "Faktor VIII-Konzentrat, hochgereinigt und in Lösung erhitzt" ARZNEIMITTEL-FORSCHUNG, Bd. 31, Nr. 4, 1981, Seiten 619-622, XP001161009
- FISCHER BE ET AL.: "von Willebrand Factor: Measuring its Antigen or Function?" THROMBOSIS RESEARCH, Bd. 91, 1998, Seiten 39-43,
- BARTHELS M & VON DEPKA M: "Das Gerinnungskompendium" 2003, GEORG THIEME VERLAG

## Beschreibung

Gegenstand der Erfindung ist ein Konzentrat eines Faktor VIII:C-haltigen von-Willebrand-Faktors, das aufgrund seiner besonderen Zusammensetzung therapeutische Vorteile aufweist.

Der funktionelle von-Willebrand-Faktor (vWF), ein Glykoprotein, zirkuliert im Blutkreislauf mit unterschiedlicher Molekulargewichtverteilung, den sog. Multimeren, wobei die Multimeren eine Molekulargewichtsverteilung von 500 KiloDalton (kD) bis hin zu 20.000 kD aufweisen können. Die kleinste Einheit ist hierbei das Dimer mit einem Molekulargewicht von ca. 550 kD; es besteht aus zwei Monomeren, die durch Disulfidbrücken miteinander verbunden sind. Aus diesen Dimeren entstehen durch weitere Disulfidverknüpfungen Polymere, sog. Multimere, mit einem Molekulargewicht von bis zu 20.000 kD. Die Molekulargewichtsverteilung der von-Willebrand Multimeren kann mittels der Elektrophorese in Agarose-Gelen sowohl quantitativ wie auch qualitativ bestimmt werden. (Lit.1,2,3). Die physiologische Funktion des von-Willebrand-Faktors ist seine Eigenschaft, am verletzten Endothel zu adhärieren und Thrombozyten zu aggregieren. (Lit.4) Dadurch kommt es in der sog. primären Hämostase zunächst zur Bildung eines Thrombozytenpfropfes und somit zu einer ersten Blutstillung; im Weiteren kommt es zum Ablauf der Gerinnungskaskade, der sog. plasmatischen Hämostase, und schließlich zum Wundverschluss.

Eine weitere wichtige Funktion des von-Willebrand-Faktors ist seine Fähigkeit, Komplexe mit Faktor VIII:C (FVIII:C) zu bilden; durch diese Komplexbildung mit dem vWF wird der FVIII:C im Plasma vor proteolytischem Abbau geschützt. Bei einem gesunden Organismus ist immer ein ausreichend hohes Niveau an FVIII:C im Komplex mit vWF vorhanden. Es wird hierbei davon ausgegangen, dass die beiden Proteine FVIII:C und vWF durch eine nicht-kovalente Bindung zwischen dem N-Terminus der leichten Kette von FVIII:C und dem N-Terminus der vWF Untereinheit verbunden sind. ( Lit.5,6)

Beobachten konnte man derartige Zusammenhänge z.B. bei der Substitution von von Willebrand Patienten mit Kryopräzipitat aus dem Plasma gesunder Spender. In Patienten induzierte die Substitution mit normalem Kryopräzipitat einen Anstieg von FVIII:C über mehrere Stunden. Der Abfall erfolgte langsam mit der Halbwertszeit des vWF.

Der antihämophile Faktor, das Faktor VIII Gerinnungsprotein (FVIII:C), zirkuliert im Plasma zusammen mit dem vWF als nicht-kovalent gebundener Komplex, wobei der vWF/FVIII:C-Komplex nur schwer präparativ aufzutrennen ist. Lange Zeit war nicht bekannt, dass vWF und FVIII:C zwei unterschiedliche Proteine sind, die im Organismus auch an unterschiedlichen Stellen synthetisiert werden: der FVIII:C in der Leber, der vWF in Endothelzellen und Megakaryozyten. (Lit. 9,10).

Das Fehlen des vWF oder auch nur dessen Verminderung hat eine verlängerte Blutungszeit und eine gravierende Blutungsneigung zur Folge; das Krankheitsbild wird von-Willebrand-Syndrom genannt und kann in mehreren Erscheinungsformen auftreten. Es erstreckt sich hierbei von einer abnormen Größenverteilung der vWF-Multimeren bis hin zum teilweisen oder gänzlichen Fehlen eines funktionellen von-Willebrand Faktors, dem sog. Typ III des von-Willebrand-Syndroms. Es können hierbei sowohl die hochmolekularen als auch die niedermolekularen Multimeren vermindert sein oder sogar gänzlich fehlen. Das von-Willebrand-Syndrom ( z.B. Typ III) hat auch einen Mangel an FVIII:C und damit eine Hämophilie A zur Folge, da FVIII:C in einem Komplex mit vWF geschützt wird und ohne diesen in kürzester Zeit im Plasma proteolytisch abgebaut wird.

Bei der Substitution eines Hämophilie-Patienten mit normalem Kryopräzipitat beobachtet man nur einen raschen und kurzen Anstieg messbarer FVIII:C Aktivität. (Lit,7,8). Substituiert man jedoch einen von Willebrand Patienten mit Plasma oder Kryopräzipitat eines hämophilen Spenders, so misst man paradoxerweise auch einen FVIII-Anstieg. Dies ist mit der Stabilisierung durch den vWF zu erklären. Der von Willebrand Patient synthetisiert FVIII, der aber nach Freisetzung ins Plasma fortwährend proteolytisch gespalten wird.

Der vWF ist für die normale Blutgerinnung zusammen mit dem FVIII:C von großer Bedeutung. Die Konzentration von vWF im Plasma beträgt ca. 10 µg/ml. FVIII:C stellt von der Masse her einen viel geringeren Proteinanteil dar, ca. 0,2 µg/ml.

Der vWF vermittelt, wie anfangs erwähnt, die Thrombozytenaggregation und somit die primäre Hämostase an den verletzten Gefäßen. Zusammen mit anderen gerinnungsaktiven Faktoren wie den sog. Kontaktfaktoren, FVIII:C, Phospholipiden und Calcium erfolgt über die Aktivierung von Faktor X die weitere Blutgerinnung bis zu Bildung von Fibrin und der Wundverschluss. (Lit. 11)

Der vWF/FVIII:C Komplex ist nur schwer zu trennen, deshalb findet sich der FVIII:C zusammen mit vWF im Kryopräzipitat, bzw. eluiert bei Gelfiltrationen zusammen mit vWF im Ausschlussvolumen. (Lit. 12)

Zur Anreicherung des vWF/FVIII:C wird in der Plasmaindustrie häufig das Verfahren der Kryopräzipitation eingesetzt. Hierbei entsteht durch gezieltes Auftauen von tiefgefrorenem Plasma ein unlöslicher Niederschlag (Kryopräzipitat). Trennt man diesen Niederschlag ab, so erhält man neben dem Kryopräzipitat das sog. kryoarme Plasma. Im Kryopräzipitat findet sich angereichert der vWF/FVIII:C-Komplex zusammen mit einem Teil der Plasmaproteine Fibrinogen und Fibronectin. Das Multimerenspektrum des vWF im Kryopräzipitat enthält eine wirksame Zusammensetzung der Multimeren, vergleichbar der vom normalen Plasma. Es präzipitieren hierbei vorwiegend die höhermolekularen Multimeren, wobei nahezu alle hochmolekularen Multimeren des vWF aus dem Plasma im Kryopräzipitat wiedergefunden werden. Etwa 20% des vWF, der keine messbare Aktivität aufweist, verbleibt zusammen mit FVIII:C im kryoarmen Plasma.

Ein Plasmapool gesunder Menschen enthält "per definitionem" 1 IE/ml funktionelle Aktivität bezogen auf alle Gerinnungsfaktoren. Die funktionelle Aktivität des vWF wird gewöhnlich durch die Ristocetin vermittelte Thrombozytenaggregation gemessen, die in Korrelation mit der Konzentration des intakten vWF steht. Man beschreibt diese als vWF-Ristocetin-Cofaktor-Aktivität (vWF:RCoF) mit Konzentrationsangaben in IE/ml. Das zugeordnete Protein bezeichnet man als vWF-Antigen, abgekürzt als vWF:Ag.

Bei hochgradigen von-Willebrand-Erkrankungen ist die Substitution mit einem vWF-Konzentrat, das einen hohen funktionellen Anteil an FVIII:C aufweist, von erheblichem Vorteil; zum einen ist der gebundene gerinnungsaktive Faktor VIII:C ein Maß für die Bindekapazität und signalisiert einen intakten vWF, zum anderen führt das Vorhandensein von FVIII:C zu einer signifikanten Verkürzung der Blutungszeit. Ohne FVIII:C wäre es erforderlich, bei der Behandlung von von-Willebrand-Erkrankungen durch Substitution zusätzlich FVIII:C-Präparate zu applizieren. Voraussetzung für die schnelle Wirksamkeit bei von-Willebrand-Erkrankungen ist also ein vWF mit normaler Faktor VIII:C Bindekapazität, vorteilhaft angereichert mit einem hochmolekularen Multimerenanteil an vWF und einem Anteil an intaktem Faktor VIII:C.

Die europäische Patentanmeldung EP 0 705 846 (Lit.13) beschreibt ein präparatives Verfahren zur Trennung des von-Willebrand Faktors in eine höhermolekulare Fraktion und eine niedermolekulare Fraktion des vWF. Diese Trennung wird erreicht, indem der vWF auf einem Affinitätsträger gebunden und dann bei unterschiedlicher Salzkonzentration von diesem eluiert wird. Auf diese Weise lassen sich hochmolekulare vWF-Fraktionen, die eine besonders hohe physiologische Aktivität haben, erhalten.

Es sind auch schon chromatographische Verfahren zur Auftrennung des vWF in höhermolekulare und niedermolekulare Multimere bekannt. Hierbei war es jedoch nicht möglich, optimale vWF/FVIII:C-Komplexe mit angereicherten hochmolekularen Multimeren des vWF gezielt zu erhalten.

Es ist auch bekannt, dass F VIII:C, sei es nun rekombinanter oder plasmatischer FVIII bei wiederholter Gabe und in höheren Konzentrationen, dissoziiert vom von Willebrand Faktor, zu unerwünschten Immunreaktionen führen kann, da die Antikörperbildung je nach Herstellungsart und Reinheit unterschiedlich stark induziert werden kann. Diese Antikörper, sog. Hämophilie A Hemmkörper oder FVIII:C Inhibitoren, die in "Bethesda-Einheiten" gemäß einer Publikation: Thrombos, Diathes haemorrh. (Stuttg.), 1975, 34, 869. (Lit.14) quantitativ bestimmt werden können, führen zu unerwünschten Nebenwirkungen und gegebenenfalls zu Blutungen.

Werden diese FVIII:C Präparate mit multimerem von Willebrand Faktor vorinkubiert, bleibt die Bildung dieser anti-FVIII-Immunglobuline weitestgehend aus und man kann ohne diese Nebenwirkungen befürchten zu müssen, wiederholt größere Mengen anwenden. Das erfindungsgemäße Präparat hat somit einen bedeutenden Anwendungsvorteil. Nachweislich wurde dieser Zusammenhang in einem Haemophilie Maus Modell gefunden. (Lit.15).

Heimburger N et al. (1981) Arzneimittelforschung 31: 619-622, beschreiben Konzentrate von Faktor VIII, die von-Willebrand Faktor enthalten (vWF oder Faktor VIII RAg), wobei gepooltes Kryopräzipitat über eine Aluminiumhydroxid-Adsorption und durch Zugabe von Glycin vorgereinigt wird, wobei bei der fraktionierten Fällung mit Glycin Fibrinogen abgetrennt wird. Aus dem Glycin-Überstand wird über eine Kochsalz-Fällung der Faktor VIII/vWF-Komplex abgetrennt, mit Saccharose und Glycin stabilisiert und 10 Stunden bei 60°C pasteurisiert.

US 5288853 offenbart ein Verfahren zur Reinigung von Faktor VIII /vWF-Komplex, wobei der Komplex aus einer Flüssigkeit mittels Gycin/NaCl präzipitiert wird, wobei Glycin in einer Konzentration von 1,5-2,5 M und NaCl in einer Konzentration von 1-2 M vorliegt.

Der im Patentanspruch 1 angegebenen Erfindung liegt das Problem zugrunde, ein Konzentrat eines Faktor VIII:C-haltigen von-Willebrand-Faktors herzustellen, das angereicherte hochmolekulare Multimere des vWF enthält und ein Verhältnis der vWF:RCoF-Aktivität zu vWF:Ag größer als 1 aufweist.

Das vorstehend beschriebene Problem wird durch das im Patentanspruch 1 genannte Konzentrat gelöst. Dieses Konzentrat wird durch fraktionierte Ausfällung aus einer den Faktor VIII:C und den von-Willebrand-Faktor enthaltenden Flüssigkeit gewonnen und weist einen erhöhten Gehalt an hochmolekularen Multimeren des von-Willebrand-Faktors und ein Verhältnis der vWF:RCoF-Aktivität zu vWF:Ag größer als 1 auf.

Die mit der Erfindung erzielten Vorteile bestehen darin, dass ein Konzentrat eines Faktor VIII:C-haltigen von-Willebrand-Faktors zur Verfügung gestellt werden kann, das durch eine einfache präparative fraktionierte Ausfällung aus einer den Faktor VIII:C und den von-Willebrand-Faktor enthaltenden Flüssigkeit gewonnen werden kann, wobei das Konzentrat einen erhöhten Gehalt an hochmolekularen Multimeren des von-Willebrand-Faktors und ein Verhältnis der vWF:RCoF-Aktivität zu vWF:Ag größer als 1 aufweist. Das so erhaltene Konzentrat eignet sich zur Substitution bei hochgradigen von-Willebrand Erkrankungen; das Vorhandensein des Faktor VIII:C ist hierbei von erheblicher Bedeutung, denn der gebundene gerinnungsaktive Faktor VIII:C wird von vWF stabilisiert und führt so zu einer signifikanten Verkürzung der Blutungszeit. Der hohe Anteil an hochmolekularen Multimeren ist eine wesentliche Voraussetzung für dessen schnelle Wirksamkeit.

Weitere vorteilhafte Ausgestaltungen der Erfindung sind in den Patentansprüchen 2 und folgende angegeben.

Das erfindungsgemäße Konzentrat kann aus humanem Plasma, einer Plasmafraktion,wie z.B. Kryopräzipitat oder aus gentechnisch modifiziertem Zellmaterial gewonnen werden. Bevorzugtes Ausgangsmaterial hierfür ist humanes Kryopräzipitat, das den vWF-FVIII:C-Komplex neben den Plasmaproteinen Fibrinogen und Fibronektin enthält. Gewonnen wird dieses Kryopräzipitat aus tiefgefrorenem Citratplasma, das durch gezieltes Erwärmen (Tempern) in den Flüssigzustand überführt wird, wobei bei Temperaturen zwischen 0 und +2°C ein Teil des Fibrinogens, des Fibronektins und der vWF:FVIII:C-Komplex als Präzipitat zurückbleiben und z.B. durch Zentrifugation abgetrennt werden können. Das so gewonnene Kryopräzipitat kann tiefgefroren zwischengelagert werden und dient als Ausgangsmaterial zur Gewinnung des gereinigten vWF/FVIII:C-Komplexes.

Das erfindungsgemäße Konzentrat wird vorzugsweise durch fraktionierte Ausfällung mittels Glycin und Natriumchlorid gewonnen. Hierbei wird eine wässrige Lösung von Kryopräzipitat unter Rühren mit Glycin versetzt bis Fibrinogen weitestgehend aus der Lösung ausgefallen ist. Der ausgefällte Fibrinogenrückstand wird anschließend durch Zentrifugation abgetrennt. Aus dem Überstand wird unter Rühren durch Zugabe eines Alkali- oder Erdalkalisalzes, in einer bevorzugten Ausgestaltung durch die Zugabe von Natriumchlorid, der Komplex vWF/FVIII:C ausgefällt und durch Zentrifugation abgetrennt. Das hierbei erhaltene vWF/FVIII:C-haltige Präzipitat wird mit einem isotonischen Puffer gelöst, mit Saccharose und Glycin stabilisiert und anschließend pasteurisiert.

Im erfindungsgemäßen Verfahren wird die Fällung des vWF/FVIII:C-haltigen Präzipitats mit Konzentrationen von 0,93 bis 2,13 mol/l (70 bis 160 g/l) Glycin und von 1,17 bis 2,74 mol/l (100 bis 160 g/l) Natriumchlorid durchgeführt. Durch diese Einstellung eines Konzentrationsbereichs kann die Aktivität/Ratio zugunsten einer höheren vWF:RCoF-Aktivität verschoben werden, was mit einer Anreicherung hochmolekularer vWF-Multimeren einhergeht.

Durch die Einstellung eines bestimmten Konzentrationsbereiches vom Fällungsmittel Glycin bei einer Konzentration von 1,17 bis 2,74 mol/l Natriumchlorid kann die Aktivitäts-Ratio zu Gunsten einer höheren vWF:RCoF-Aktivität verschoben werden, was ein Ergebnis der Anreicherung hochmolekularer vWF-Multimeren ist. Eine Schlüsselrolle nimmt dabei die Konzentration des Glycins ein, was auch Gegenstand dieser Erfindung ist.

Das erfindungsgemäße Verfahren wird zweckmäßigerweise so durchgeführt, dass zunächst das gelöste Kryopräzipitat zur Adsorption des in geringen Mengen eingeschlossenen Prothrombin-Komplexes mit einer Aluminiumhydroxidsuspension versetzt, dann gerührt und abgetrennt wird. Der Überstand enthält dann den Faktor VIII:C und den von-Willebrand-Faktor, welche durch fraktionierte Ausfällung gewonnen werden.

Das Kryopräzipitat wird unter Rühren und leichtem Erwärmen in einem isotonischen Puffer gelöst, so dass man eine Proteinkonzentration von 2 bis 3% erhält. Die so gewonnene Rohkryolösung wird dann zur Adsorption der Prothrombinkomplex-Faktoren mit einer Aluminiumhydroxid-Suspension versetzt und unter Rühren die restlichen Prothrombinfaktoren adsorbiert und zusammen mit dem Al(OH)₃-Pellet abgetrennt. Nach Entfernung der Prothrombinkomplex-Faktoren kann die Kryolösung einer Virusinaktivierung durch Pasteurisierung oder mit Acridin oder Acridinderivaten in Übereinstimmung mit der DE 44 44 045 unterworfen und stabilisiert werden, wozu sich Calciumionen besonders gut eignen.

Die nach der Virusinaktivierung erhaltene Kryolösung wird anschließend unter Rühren mit Glycin versetzt, das Fibrinogen präzipitiert und durch Zentrifugation abgetrennt. Dem erhaltenen Überstand wird anschließend unter Rühren NaCl zugegeben und so der vWF/FVIII:C ausgefällt und durch anschließende Zentrifugation abgetrennt. Das erhaltene vWF/FVIII:C -haltige Präzipitat wird in einem isotonischen Puffer gelöst, mit Saccharose und Glycin stabilisiert und anschließend 10 Stunden auf 60°C erhitzt. Nach erfolgter Pasteurisierung dient die erhaltene Lösung als Ausgangsmaterial zur Gewinnung des vWF/FVIII:C-Komplexes mit angereichertem, hochmolekularem Multimeranteil.

Ausführungsbeispiele der Erfindungen sind in den Beispielen 1 bis 6 dargestellt.

### Beispiel 1

### Lösen von Kryopräzipitat, Al(OH)₃-Adsorption und Fibrinogenabtrennung

200 g Kryopräzipitat wurden zerkleinert mit einer 0,1 m NaCl-Glycin-Lösung in 800 ml aufgelöst. Die Kryolösung wurde mit 10 Vol% einer 1,5%-igen Al(OH)₃ Suspension versetzt, 15 min. gerührt und abzentrifugiert. Das abgetrennte Al(OH)₃-Pellet wurde verworfen. Der Al(OH)₃-Überstand (820 ml) wurde unter Rühren mit Glycin versetzt, bis das Fibrinogen aus der Lösung abgeschieden war. Das Präzipitat wurde abzentrifugiert, der vWF/FVIII:C-Komplex haltige Überstand wurde weiterverarbeitet.

### vWF/FVIII:C-Komplex Ausfällung, Lösen, Stabilisieren, Pasteurisieren

Der glycinhaltige Überstand wurde unter Rühren mit 15% NaCl versetzt und der vWF/FVIII:C-Komplex quantitativ ausgefällt. Das Präzipitat wurde in 64 ml NaCI/Glycin Puffer aufgelöst, mit Saccharose (1 g/ml) und Glycin (150 g/l) stabilisiert und 10 Std. bei 60°C pasteurisiert. Nach dem Erkalten wurde die pasteurisierte Lösung mit dem gleichen Volumen Glycin/NaCl Puffer verdünnt.

### Ausfällen der vWF/FVIII:C-Fraktion mit erhöhtem Anteil an hochmolekularen Multimeren

Die verdünnte Lösung (220 ml), die 1,6 g/l NaCl und 124,4 g/l Glycin enthielt, wurde in 3 Ansätzen a,b,c, mit je 0,75 Teilen eines Fällmediums unter Rühren versetzt, so dass der Fällungsansatz
a) 80 g/l
b) 90 g/l
c) 100 g/l

Glycin enthielt, und die Endkonzentration an NaCl in allen Fällen 122 g/l erreichte. Dabei entstand jeweils ein feines Präzipitat, das nach ca. 45 min. Rühren abzentrifugiert wurde. Die in einem isotonischen Puffer gelöste Fraktion (je 44 ml) enthielt nun den vWF und FVIII:C, angereichert mit hochmolekularen Multimeren, was in der nachfolgenden Tabelle 1 in Verhältniszahlen zum Ausdruck kommt. Die Auswertung nach vWF:RCoF-Aktivität, vWF:Ag und FVIII:C ergab folgende Ratio:

**Tabelle 1: Ratios FVIII:C, vWF:RCoF, vWF:Ag aus den Ansätzen gemäß Beispiel 1.**

| | **FVIII:C zu vWF:RCoF** | **FVIII:C zu vWF:Ag** | **vWF:Ag zu vWF:RCoF** |
|---|---|---|---|
| Ausgangsmaterial: | 1 : 3,1 | 1 : 2,5 | 1 : 1,2 |
| Ansatz a) | 1 : 2,4 | 1 : 0,7 | 1 : 3,6 |
| Ansatz b) | 1 : 3,1 | 1 : 1,3 | 1 : 2,4 |
| Ansatz c) | 1 : 3,5 | 1 : 1,8 | 1 : 2,4 |
| Kryollösung nach Al(OH)₃Ads. (Referenz) | 1 : 1,6 | 1 : 2,6 | 1 : 0,6 |

Tabelle 1 zeigt, dass die Ratio von vWF:Ag zu vWF:RCoF im Ausgangsmaterial nahe 1 war. Bei Ansatz a) bis c) war die Ratio zu Gunsten vWF:RCoF-Aktivität im Vergleich zum vWF:Ag bis zu verdreifacht.

### Beispiel 2

Ausgangsmaterial für die Gewinnung einer Fraktion mit angereicherten hochmolekularen Multimeren war hier eine vorfraktionierte, pasteurisierte vWF/FVIII:C-haltige Lösung, gewonnen aus Kryopräzipitat. Die Lösung war klar und homogen und enthielt in dieser Verfahrensstufe 1,6 g/l NaCl und 124,4 g/l Glycin.

Zur Fällung eines ersten Präzipitats des vWF/FVIII:C-Komplexes wurde erfindungsgemäß in mehreren Ansätzen das Gleichgewicht NaCl-Glycin durch Zugabe eines jeweils abgestimmten Fällmediums so eingestellt, dass bevorzugt die hochmolekularen vWF-Multimeren ausfielen, was durch das Verhältnis der Konzentrationen der vWF:RCoF-Aktivität zum vWF:Ag kontrolliert werden konnte und deutlich größer als 1 war.

Nachdem das erste Präzipitat abzentrifugiert war, wurde im Überstand jeweils die Glycinkonzentration erhöht (Nachfällung) und der im Überstand verbliebene vWF auch noch ausgefällt. In diesem 2. Präzipitat war der hochmolekulare Anteil deutlich vermindert, was aus der Abnahme des Verhältnisses der vWF:RCoF-Aktivität zur Antigenkonzentration ersichtlich war.

### Beschreibung von 3 Fällungsansätzen A, B, C:

Je 200 ml Ausgangsmaterial wurde mit dem 0,75-fachen Volumen eines Fällmediums (150 ml) unter Rühren bis zur vollständigen Zugabe versetzt. Es entstand ein feines Präzipitat, das abzentrifugiert wurde.

200 ml vWF/FVIII:C-haltiges Ausgangsmaterial, enthielt bereits folgende Konzentrationen an NaCl und Glycin:
1,6 g/l NaCl, 124,4 g/l Glycin:

Zur Erzielung des 1. Präzipitates hatten die Fällmedien folgende Konzentrationen an NaCl und Glycin:

| | | |
|---|---|---|
| Fällmedium für Ansatz A: | 283 g/l NaCl | kein Glycin |
| Fällmedium für Ansatz B: | 283 g/l NaCl | 45 g/l Glycin |
| Fällmedium für Ansatz C: | 283 g/l NaCl | 90 g/l Glycin |

Zu je 200 ml Ausgangsmaterial wurde im Ansatz A bis C 150 ml entsprechendes Fällmedium zugeben, dabei entstanden folgende Endkonzentrationen an NaCl und Glycin im jeweiligen Fällungsansatz:

| | NaCl | Glycin |
|---|---|---|
| Ansatz A | 122,2 g/l | 71,1 g/l |
| Ansatz B | 122,2 g/l | 90,4 g/l |
| Ansatz C | 122,2 g/l | 109,6 g/l |

Die entstandenen Präzipitate wurden abgetrennt und eingelöst. Von den gelösten Präzipitaten (∼ 42 ml) wurden die Konzentrationen vWF:RCoF, vWF:Ag und FVIII:C bestimmt und in nachfolgender Tabelle 2 dargestellt.

**Tabelle 2**

| | **FVIII:C [IE/ml]** | **vWF:RCoF [IE/ml]** | **vWF:Ag [IE/ml]** |
|---|---|---|---|
| Ausgangsmaterial | 14,7 | 39,8 | 35,0 |
| Ansatz A | 11,2 | 24,2 | 8,2 |
| Ansatz B | 44,7 | 138,7 | 87,2 |
| Ansatz C | 46,3 | 150,3 | 113,7 |

Die jeweilige Ratio der Aktivitäten aus Tabelle 2 wurde errechnet und in Tabelle 3 dargestellt.

**Tabelle 3: Ratio FVIII:C, vWF:RCoF, vWF:Ag aus den Ansätzen gemäß Beispiel 2.**

| | **FVIII:C zu vWF:RCoF** | **FVIII:C zu vWF:Ag** | **vWF:Ag zu vWF:RCoF** |
|---|---|---|---|
| Ausgangsmaterial: | 1 : 2,7 | 1 : 2,4 | 1 : 1,1 |
| Ansatz A | 1 : 2,2 | 1 : 0,7 | 1 : 3,0 |
| Ansatz B | 1 : 3,1 | 1 : 2,0 | 1 : 1,6 |
| Ansatz C | 1 : 3,5 | 1 : 2,5 | 1 : 1,3 |

Die in den Ansätzen A bis C erzielte Ratio vWF:Ag zu vWF:RCoF-Aktivität war im Vergleich zum Ausgangsmaterial zu Gunsten der vWF:RCoF-Aktivität verschoben, was eine Zunahme der hochmolekularen Multimeren bedeutete.

### Präzipitat oder Nachfällung aus den Überständen der Ansätze A bis C:

Die Überstände der Ansätze A bis C wurden unter Rühren mit Glycin versetzt, und zwar so, dass alle 3 Ansätze eine Glycinkonzentration von je 160 g/l erreichten. Die entstandenen Präzipitate wurden abzentrifugiert und eingelöst.

Nach der Bestimmung von vWF:RCoF, vWF:Ag und FVIII:C-Konzentration wurden die Verhältniszahlen errechnet. Die Ratio wurde in Tabelle 4 dargestellt.

**Tabelle 4: Ratios FVIII:C, vWF:RCoF, vWF:Ag aus den Ansätzen gemäß Beispiel 2.**

| | **FVIII:C zu vWF:RCoF** | **FVIII:C zu vWF:Ag** | **vWF:Ag zu vWF:RCoF** |
|---|---|---|---|
| Nachfällung Ansatz A | 1 : 4,5 | 1 : 3,7 | 1 : 1,1 |
| Nachfällung Ansatz B | 1 : 7,65 | 1 : 12,1 | 1 : 0,63 |
| Nachfällung Ansatz C | 1 : 5,9 | 1 : 15,4 | 1 : 0,26 |

Die Nachfällungen, dargestellt in Tabelle 4, zeigten in der Ratio: vWF:Ag zu vWF:RCoF eine deutliche Verminderung der vWF:RCoF-Aktivität, was auf eine verminderte Menge hochmolekularer Multimere hindeutete und somit auch auf eine Verminderung der vWF-Funktionalität.

### Beispiel 3

Wie in Beispiel 2 wurde eine vorfraktionierte und pasteurisierte vWF und FVIII:C-haltige völlig klare Lösung eingesetzt, die in dieser Präparationsstufe 1,6 g/l NaCl und 124,4 g/l Glycin enthielt. In 4 Fällungsansätzen, mit jeweils gleicher NaCl-Glycinkonzentration, wurde hier die Zugabe und Inkubationszeit variiert.

Die Glycinkonzentration war im Vergleich zu Beispiel 1 und Beispiel 2 im Fällungsansatz höher; Fällungsansätze 1 bis 4 unterschieden sich nur in der Zugabe- und Inkubationszeit, um zu klären und zu belegen, dass die bei der Fällung entstehende Ratio vWF:Ag/vWF:RCoF nicht von den Einwirkzeiten, sondern in erster Linie von der Glycin-Konzentration abhing.

Bei den Ansätzen 1 bis 4 wurden je 200 ml Ausgangsmaterial unter Rühren mit je 150 ml Fällmedium versetzt, das 283,01 g/l NaCl und 133,58 g/l Glycin enthielt. Die Variablen waren die Zugabe- und Inkubationszeit, NaCl- und Glycinkonzentration waren bei allen Ansätzen gleich, wie aus Tabelle 5 ersichtlich ist.

**Tabelle 5: Zugabe- und Inkubationszeiten im Fällungsansatz Beispiel 3.**

| | **Zugabe [min]** | **Inkubation [min]** | **NaCl-Konz. [g/l]** | **Glycin-Konz. [g/l]** |
|---|---|---|---|---|
| Ansatz 1: | 120 | 30 | 122,2 | 128,3 |
| Ansatz 2: | 60 | 60 | 122,2 | 128,3 |
| Ansatz 3: | 60 | 90 | 122,2 | 128,3 |
| Ansatz 4: | 60 | 240 | 122,2 | 128,3 |

Die entstandenen Präzipitate wurden abzentrifugiert und eingelöst. Der Überstand von Ansatz 1 wurde mit weiteren kristallinen Glycin, auf eine Konzentration von 160 g/l eingestellt und 2 Std. gerührt. Das entstandene Präzipitat wurde ebenfalls eingelöst. Die Aktivitätsgehalte an FVIII:C, vWF:RCoF sowie vWF:Ag wurden gemessen und die Ratio zueinander berechnet. Sie sind in Tabelle 6 dargestellt.

**Tabelle 6: Ratios FVIII:C, vWF:RCoF, vWF:Ag aus den Ansätzen gemäß Beispiel 3; Ratios FVIII:C, vWF:RCoF, vWF:Ag.**

| | **FVIII:C zu vWF:RCoF** | **FVIII:C zu vWF:Ag** | **vWF:Ag zu vWF:RCoF** |
|---|---|---|---|
| Ansatz 1 | 1 : 2,4 | 1 : 2,7 | 1 : 0,9 |
| Nachfällung Glycin Aus Ansatz 1 | 1 : 1,4 | 1 : 5,3 | 1 : 0,3 |
| Ansatz 2 | 1 : 2,1 | 1 : 2,9 | 1 : 0,7 |
| Ansatz 3 | 1 : 2,3 | 1 : 2,7 | 1 : 0.9 |
| Ansatz 4 | 1 : 2,3 | 1 : 3,0 | 1 : 0,8 |

Hier zeigte sich, dass die Ansätze 1 bis 4 trotz unterschiedlicher Zugabe- und Inkubationszeiten in den Ratios der gemessenen Aktivitäten nahezu vergleichbar waren. Bei allen 4 Ansätzen war die NaCl-Konzentration und die Glycinkonzentration gleich.

Nur die Nachfällung von Ansatz 1 hob sich deutlich ab: Der vWF-Ristocetin-Cofaktorgehalt war deutlich verringert, der vWF:Ag-Gehalt war stark erhöht. In der gelösten Nachfällung fehlten eindeutig die hochmolekularen Multimeren (ohne Abbildung).

### Beispiel 4

Ausgangsmaterial war hier ebenfalls eine vorgereinigte vWF/FVIII:C-Fraktion, die 1,6 g/l NaCl und 124,4 g/l Glycin enthielt
Ansatz 1: Hier wurden 200 ml Ausgangsmaterial vorgelegt und unter Rühren wurde 150 ml Fällmedium zugegeben, das 283,01 g/l NaCl und 133,5 g/l Glycin enthielt. Es wurde gerührt bis die Fällung vollständig war, dann wurde das Präzipitat abzentrifugiert, aufgelöst und die Aktivität wurde gemessen.
Ansatz 1a: Der verbleibende Überstand wurde unter Rühren mit Glycin weiterversetzt (nachgefällt), bis die Konzentration von 160 g/l erreicht war, das Präzipitat der Nachfällung wurde abzentrifugiert und gelöst, die Aktivität wie bei Ansatz 1 gemessen.
Ansatz 2: 1 Teil des gleichen Ausgangsmaterials wurde mit 1 Teil eines anderen Fällmediums versetzt, das 300 g/l NaCl enthielt und kein Glycin. Nach vollständiger Zugabe hatten wir eine Glycin-Konzentration von 66,7 g/l, die NaCl-Konzentration betrug 151,5 g/l. Das entstandene Präzipitat wurde abzentrifugiert, gelöst und die Aktivität wie bei Ansatz 1 gemessen.
Ansatz 2a: Der verbliebene Überstand von Ansatz 2 wurde ebenfalls durch Zugabe von Glycin weiter gefällt, bis eine Glycinkonzentratin von 160 g/l erreicht war; das Präzipitat wurde abzentrifugiert, gelöst und die Aktivität gemessen: (Nachfällung)

**Tabelle 7: Fällungskonzentrationen an Glycin und NaCl.**

| | | |
|---|---|---|
| | **NaCl-Konzentration im Fällungsansatz [g/l]** | **Glycin-Konzentration im Fällungsansatz [g/l]** |
| Ansatz 1: | 122,2 | 128,3 |
| Nachfällung 1a: | 122,2 | 160,0 |
| Ansatz 2: | 151,5 | 66,7 |
| Nachfällung 2a: | 151,5 | 160,0 |

Die Bestimmung von vWF:RCoF, vWF:Ag und FVIII:C ergab folgende Ratio, wie in Tabelle 8 dargestellt.

**Tabelle 8: Ratios FVIII:C, vWF:RCoF, vWF:Ag aus den Ansätzen gemäß Beispiel 4.**

| | **FVIII:C zu vWF:RCoF** | **FVIII:C zu vWF:Ag** | **vWF:Ag zu vWF:RCoF** |
|---|---|---|---|
| Ansatz 1 | 1 : 2,7 | 1 : 2,9 | 1 : 0,9 |
| Nachfällung 1a | 1 : 10,5 | 1 : 11,3 | 1 : 0,9 |
| Ansatz 2 | 1 : 2,4 | 1 : 1,8 | 1 : 1,3 |
| Nachfällung 2a | 1 : 2,6 | 1 : 4,8 | 1 : 0.6 |

Zu Tabelle 8: Ansatz 1 und Ansatz 2 zeigten für ein vWF-Konzentrat eine vorteilhafte Multimerenverteilung, bei Ansatz 2 waren die hochmolekularen Multimere noch stärker vertreten. Im Ansatz 1a und besonders im Ansatz 2a waren die hochmolekularen Anteile vermindert (ohne Abbildung).

### Beispiel 5

### Ansatz A

### Herstellung eines Konzentrats, in dem die hochmolekularen vWF-Multimeren angereichert waren:

Entsprechend Beispiel 1 wurden 3,64 kg Kryopräzipitat zu ca. 4000 ml einer verdünnten pasteurisierten Lösung, die vWF und FVIII:C enthielt, aufgearbeitet. Durchführung der Fällung zu einer Fraktion mit angereicherten hochmolekularen vWF-Multimeren und FVIII:C:

4000 ml der pasteurisierten, verdünnten vWF/FVIII:C-Lösung wurden mit 3000 ml eines Fällmediums (24,44 g NaCl, 24,15 g Glycin, 2000 ml WFI, pH 6,8) innerhalb 60 min. unter Rühren versetzt und weitere 90 min. ohne Rühren nachinkubiert. Die ausgebildete Fällung wurde in der Zentrifuge bei 6000xg für 45 min. abzentrifugiert. Das gewonnene Präzipitat (Präzipitat 1) wurde ad 400 ml mit Lösepuffer (Lösepuffer: 1,46 g NaCl, 10,14 g Glycin, 500 ml WFI, pH 7,0) gelöst.

### Stabilisierung, Endformulierung, Lyophilisation

Die entstandenen Lösung mit den angereicherten hochmolekularen vWF-Multimeren wurde mit 0,5% Human-Albumin stabilisiert und auf einen Puffergehalt von 3,5 g/l NaCl, 5,8 g/l Tri-Na-Citrat x 2H₂O 20 g/l Glycin, pH 7,0 dialysiert. Nach Dialyse wurde die Lösung für 60 min. bei 30.000xg ultrazentrifugiert. Der Überstand nach Ultrazentrifugation wurde dekantiert. Die ultrazentrifugierte Lösung wurde nun geteilt, in Teil I und Teil II.

Teil I wurde sterilfiltriert, abgefüllt und lyophilisiert.
Teil II wurde so belassen, ebenfalls abgefüllt und lyophilisiert. Bekanntlich trennen sich Keime bei hochtouriger Ultrazentrifugation zum Grossteil ab.

Zweck der Teilung war zu untersuchen, welchen Einfluss die Sterilfiltration auf die Ratio hat, bzw. ob nach Sterilfiltration das Spektrum der hochmolekularen Multimeren unverändert bleibt.

Wie später in Tabelle 9 gezeigt wird, blieb die Ratio und somit auch das hochmolekulare Multimerenspektrum erhalten, bis auf eine geringe Produktverdünnung und einen Handling Verlust.

Nach Lyophilisation und Rekonstitution mit WFI stand ein Konzentrat zur Verfügung, das im Vergleich zu vWF:Ag eine höhere vWF:RCoF-Konzentration aufwies. Dies war dem relativ hohen Anteil an hochmolekularen vWF-Multimeren zuzuordnen und stellt einen besonderen Vorteil in der vW-Syndrom-Indikation dar.

### Ansatz B

### Herstellung einer Fraktion vWF/FVIII:C enthaltendes Konzentrat, in dem die hochmolekularen Multimeren vermindert waren:

Die vorwiegend niedermolekulare Multimerenfraktion wurde zu einem späteren Zeitpunkt aus einem weiteren Präparationsansatz gewonnen. ( Ansatz B und mit Präzipitat 2 bezeichnet).

Entsprechend Beispiel 1 wurden 3,64 kg Kryopräzipitat zu ca. 4000 ml einer verdünnten pasteurisierten Lösung, die vWF und FVIII:C enthält, aufgearbeitet.

Durchführung der Fällung zu einer Fraktion mit verminderten hochmolekularen vWF-Multimeren und FVIII:C.

4000 ml der pasteurisierten, verdünnten vWF/FVIII:C-Lösung wurden mit 3000 ml eines Fällmediums (350 g NaCl, 165,1 g Glycin, 1000 ml, WFI, pH 6,8) innerhalb 60 min. unter Rühren versetzt und weitere 90 min. ohne Rühren nachinkubert. Die ausgebildete Fällung wurde in der Zentrifuge bei 6000xg für 45 min. abzentrifugiert. Das gewonnene Präzipitat wurde ad 400 ml mit Lösepuffer (Lösepuffer: 1,46 g NaCl, 10,14 g Glycin, 500 ml WFI, pH 7,0) gelöst.

Diese Fraktion ist praktisch mit Präzipitat 1 aus Ansatz A identisch und wurde bis zu einer weiteren Verwendung tiefgefroren, der sich aus diesem Ansatz ergebende Überstand diente zur Gewinnung der niedermolekularen v.WF Multimerenfraktion.

Der Überstand wurde durch Erhöhung der Glycinkonzentration weitergefällt:

Ca. 40 I Überstand wurden durch weitere Zugabe von 30 g/l Glycin innerhalb von 60 min. bei 25 ± 2°C unter Rühren und weiterer Inkubation ohne Rühren über 60 min. gefällt. Die Endkonzentrationen im Ansatz betrugen 122 g/l NaCl und 158 g/l Glycin. Die ausgebildete Fällung wurde in der Zentrifuge bei 6000xg für 60 min. abzentrifugiert. Das nun gewonnene Präzipitat ( Präzipitat 2 ) wurde ad 250 ml mit Lösepuffer (Lösepuffer: 1,46 g NaCl, 10,14 g Glycin, 500 ml WFI, pH 7,0) gelöst. Der Überstand wurde verworfen.

Die entstandene Lösung mit dem verminderten Anteil an hochmolekularen vWF-Multimeren wurde mit 0,5% Human-Albumin stabilisiert und auf einen Puffergehalt von 3,5 g/l NaCl, 5,8 g/l Tri-Na-Citrat x 2H₂O, 20 g/l Glycin, pH 7,0 dialysiert.

Nach Dialyse wurde die Lösung für 60 min. bei 30.000 xg ultrazentrifugiert. Der Überstand nach Ultrazentrifugation wurde dekantiert, sterilfiltriert und abgefüllt.

Nach Lyophilisation und Rekonstitution mit WFI stand ein Konzentrat zur Verfügung, das im Vergleich zu vWF:Ag eine niedrigere vWF:RCoF-Konzentration aufwies.

**Tabelle 9: Aktivitäten/Ratios in den rekonstituierten Konzentrationen gemäß Beispiel 5.**

| **Aktivitäten** | | | |
|---|---|---|---|
| | **FVIII:C [IE/ml]** | **vWF:RCoF [IE/ml]** | **vWF:Ag [IE/ml]** |
| Präzipitat 1 Teil I | 25,1 | 75,0 | 26,1 |
| Präzipitat 1 Teil II | 35,7 | 93,0 | 34,2 |
| Präzipitat 2 | 12,8 | 60,5 | 63,8 |
| Ratios | | | |

| | **FVIII:C zu vWF:RCoF** | **FVIII:C zu vWF:Ag** | **vWF:Ag zu vWF:RCoF** |
|---|---|---|---|
| Präzipitat 1 Teil I | 1 : 3,0 | 1 : 1,0 | 1 : 2,9 |
| Präzipitat 1 Teil II | 1 : 2,4 | 1 : 1,0 | 1 : 2,7 |
| Präzipitat 2 | 1 : 4,7 | 1 : 5,0 | 1 : 0,9 |

### Beispiel 6

Je 200 ml Kulturüberstand, der 100 IE rekombinanten FVIII:C enthielt, wurde in 3 Ansätzen (A, B, C) mit plasmatischen von-Willebrand-Faktor-Konzentrat, das 78 IE/ml vWF:RCoF, 80 IE/ml vWF:Ag und Spuren FVIII:C enthielt, versetzt. Die Kulturlösung wurde auf 1,6 g/l NaCl und 124 g/l Glycin eingestellt. Die Ansätze wurden mit je 150 ml Fällmedium folgender Glycin/NaCl-Zusammensetzung versetzt und gerührt:

| | | |
|---|---|---|
| Ansatz A | 122,2 g/l NaCl | 71,1 g/l Glycin |
| Ansatz B | 122,2g/l NaCl, | 90,4 g/l Glycin |
| Ansatz C | 122,2g/l NaCl | 109,6g/lGlycin |

Die ausgebildete Fällung wurde in der Zentrifuge bei 30.000xg für 60 min. abzentrifugiert. Das gewonnene Präzipitat wurde mit Lösepuffer (Lösepuffer: 1,46 g NaCl, 10,14 g Glycin, 500 ml WFI, pH 7,0) gelöst und analysiert. Auch hier konnte beobachtet werden, dass bei gleichem NaCl-Gehalt und niedriger Glycinkonzentration zuerst vorwiegend hochmolekulare Multimere mit FVIII:C-Aktivität ausfallen.

Hieraus war zu erkennen, dass auch im Kulturüberstand, der sowohl rekombinanten FVIII:C als auch plasmatischen vWF enthalten kann, durch entsprechende Gleichgewichtseinstellung mit NaCl und Glycin eine Verteilung der vWF Multimeren nach Größe erzielt werden kann.

### Literaturverzeichnis:

1. "Multicenter Comparison of von-Willebrand-Factor Multimer Sizing Techniques". Thrombosis and Haemostasis, F.K. Schattauer Verlag GmbH (Stuttgart) 54 (4) 873 - 877 (1985).
2. "Electroblot and Immunoperoxidase Staining for rapid Screening of the Abnormalities of the multimeric Structure of von-Willebrand-Factor in von-Willebrand's Disease". Thrombosis and Haemostasis, F.K. Schattauer Verlag GmbH (Stuttgart) 55 (2) 246 - 249 (1986).
3. "Multimeric Analysis of von-Willebrand-Factor by vertical Sodiumdodecylsulphate Agarose Gelelectrophoresis, Vacuumblotting Technology and sensitive Visualisation by Alkaline Phosphatase Anti-Alkaline Phosphatase Complex". Thrombosis Research 66, 745 - 755 (1992).
4. "Structure-Function Relationship of Human von Willebrand Factor". Blood, Vol. 70 No. 3 (September), pp 605 - 611 (1987).
5. Fass D.N.: "Factor VIII Structure and Function", Ann NY Acad.Sci 614, 76 (1991).
6. Hamer R.J., Koedam J.A., Beeser Visser NH, Bertina R.M., van Mourik J.A., Sixma J.J.: "Factor VIII binds to vWF via its M 80.000 light chain". Eur J. Biochem., 166, 37 (1987).
7. Cornu P., Larrieu M., Caen J., Bernard J.: "Transfusion Studies in vWF: Effect on bleeding Time and Factor VIII". Br. J. Haematol. 9, 189 (1963).
8. Weiss H.J., Sussmann D., Hoyer L.W.: "Stabilisation of Factor VIII in Plasma by the vWF. Studies on posttransfusion and dissociated Factor VIII in Patients with von-Willebrand's disease". J. clin. invest. 60, 390 (1977).
9. "Synthesis of von-Willebrand-Factor by cultured human endothelial Cells", Proc Natl. Acad Sci USA 71; 1906 (1974).
10. "Synthesis of Factor VIII antigen by cultured Guinea Pig Megakaryocytes", J. clin. invest. 60, 914 (1977).
11. "Therapy of von-Willebrand Disease". Seminars in Thrombosis and Hemostasis, Volume 19, No. 1 (1993).
12. Perret B.A., Furlan M., Beck E.A.: "Isolation of small molecular forms of FVIII/vWF from plasma" Haemostasis 14, pp 289 - 295 (1984).
13. Europäische Patentanmeldung EP 0 705 846 A1.
14. A More Uniform Measurement of Factor VIII Inhibitor Thrombos. Diathes. haemorrh. (Stuttg.), 1975, 34, 869
15. Von Willebrand Factor Modulates Factor VIII Immunogenicity: Comparative Study of Different Factor VIII Concentrates in a Haemophilia A Mouse Model Thromb Haemost 2002; 88: 221-9 Mathias Behrmann, John Pasi, Jean-Marie R. Saint Remy, Ronald Kotitschke, Michael Kloft

## Patentansprüche

1. Konzentrat eines Faktor VIII:C haltigen von-Willebrand Faktors, das ein Verhältnis Einheiten vWF:RCoF-Aktivität zu Einheiten vWF:Ag größer als 1 aufweist, **dadurch gekennzeichnet, dass** es durch ein Verfahren gewonnen werden kann, bei dem eine den Faktor VIII:C und den von-Willebrand-Faktor enthaltende Flüssigkeit einer fraktionierten Fällung unterworfen wird, die folgende Schritte einschließt:
1) Fällung mittels Glycin in einer Konzentration von 0,93 bis 2,13 mol/l (70 bis 160 g/l) und
2) Fällung mittels Natriumchlorid in einer Konzentration von 1,17 bis 2,74 mol/l (100 bis 160 g/l).

2. Konzentrat nach Anspruch 1, **dadurch gekennzeichnet, dass** es aus humanem Plasma, einer Plasmafraktion vorzugsweise Kryopräzipitat oder aus gentechnologisch modifiziertem Zellmaterial gewonnen worden ist.

3. Arzneimittel zur Behandlung der Hämophilie A und des von-Willebrand Syndroms, **dadurch gekennzeichnet, dass** es ein Konzentrat der Ansprüche 1 bis 2 enthält.

4. Arzneimittel nach Anspruch 3, **dadurch gekennzeichnet, dass** es als Stabilisator Calcium-Ionen enthält.

5. Arzneimittel nach den Ansprüchen 3 und 4, **dadurch gekennzeichnet,** die Fällung (1) mittels Glycin in einer Konzentration von 0,93 bis 1,46 mol/l (70 bis 110 g/l) durchgeführt wird

6. Verfahren zur Herstellung eines Konzentrats eines Faktor VIII:C haltigen von-Willebrand Faktors, **dadurch gekennzeichnet, dass** eine den Faktor VIII:C und den von-Willebrand Faktor enthaltende Flüssigkeit einer fraktionierten Fällung unterworfen wird, die folgende Schritte einschließt:
1) Fällung mittels Glycin in einer Konzentration von 0,93 bis 2,13 mol/l (70 bis 160 g/l) und
2) Fällung mittels Natriumchlorid in einer Konzentration von 1,17 bis 2,74 mol/l (100 bis 160 g/l).

7. Verfahren nach Anspruch 6, **dadurch gekennzeichnet, dass** durch das Einstellen der Konzentrationen von Glycin und NaCl die Menge der Multimeren des von-Willebrand Faktors und damit auch die Ristocetincofaktor-Aktivität im Konzentrat gesteuert wird, wobei bei niedrigen Konzentrationen höhermolekulare Multimere und bei höheren Konzentrationen niedermolekulare Multimere bevorzugt präzipitieren.

8. Verfahren nach den Ansprüchen 6 bis 7, **dadurch gekennzeichnet, dass** das Konzentrat oder ein bei seiner Herstellung anfallendes Vorstufenprodukt stabilisiert und pasteurisiert wird.

9. Verfahren nach den Ansprüchen 6 bis 8, **dadurch gekennzeichnet, dass** ein gelöstes Kryopräzipitat zur Absorption des in geringen Mengen eingeschlossenen Prothrombin-Komplexes mit einer Aluminiumhydroxid-Suspension versetzt, dann gerührt und abgetrennt wird, das Fibrinogen mit Glycin präzipitiert und abgetrennt und anschließend der vWF:FVIII:C-Komplex ausgefällt wird, welcher nach Auflösen, Stabilisierung und Pasteurisierung einer fraktionierten Fällung gemäß Anspruch 6 unterworfen wird.

## Claims

1. Concentrate of a factor VIII:C-containing von Willebrand factor, which has a ratio of units of vWF:RCoF activity to units of vWF:Ag of greater than 1, **characterized in that** it can be obtained by a method in which a liquid which comprises factor VIII:C and von Willebrand factor is subjected to a fractional precipitation, which includes the following steps:
1) precipitation using glycine in a concentration of from 0.93 to 2.13 mol/l (70 to 160 g/l) and
2) precipitation using sodium chloride in a concentration of from 1.17 to 2.74 mol/l (100 to 160 g/l).

2. Concentrate according to Claim 1, **characterized in that** it has been obtained from human plasma, a plasma fraction, preferably cryoprecipitate, or from genetically modified cell material.

3. Medicament for the treatment of haemophilia A and of von Willebrand syndrome, **characterized in that** it comprises a concentrate of Claims 1 to 2.

4. Medicament according to Claim 3, **characterized in that** it comprises calcium ions as stabilizer.

5. Medicament according to Claims 3 and 4, **characterized in that** the precipitation (1) is carried out using glycine in a concentration of from 0.93 to 1.46 mol/l (70 to 110 g/l).

6. Process for producing a concentrate of a factor VIII:C-containing von Willebrand factor, **characterized in that** a liquid which comprises factor VIII:C and von Willebrand factor is subjected to a fractional precipitation, which includes the following steps:
1) precipitation using glycine in a concentration of from 0.93 to 2.13 mol/l (70 to 160 g/l) and
2) precipitation using sodium chloride in a concentration of from 1.17 to 2.74 mol/l (100 to 160 g/l).

7. Process according to Claim 6, **characterized in that** the quantity of the multimers of von Willebrand factor and thus also the ristocetin cofactor activity in the concentrate is controlled by adjusting the concentrations of glycine and NaCl, with preferential precipitation of higher molecular weight multimers at low concentrations and of low molecular weight multimers at higher concentrations.

8. Process according to Claims 6 to 7, **characterized in that** the concentrate or a precursor product resulting during its production is stabilized and pasteurized.

9. Process according to Claims 6 to 8, **characterized in that** a dissolved cryoprecipitate is mixed with an aluminium hydroxide suspension to absorb the prothrombin complex which is trapped in small quantities, followed by stirring and removal, the fibrinogen is precipitated with glycine and removed, and subsequently the vWF:FVIII:C complex is precipitated and, after dissolving, stabilizing and pasteurizing, is subjected to a fractional precipitation according to Claim 6.

## Revendications

1. Concentré d'un facteur de von Willebrand contenant un facteur VIII:C, qui présente un rapport des unités d'activité de vWF:RCoF aux unités de vWF:Ag supérieur à 1, **caractérisé en ce qu'**il est possible de l'obtenir par un procédé consistant à soumettre un liquide contenant le facteur VIII:C et le facteur de von Willebrand à une précipitation fractionnée, laquelle comprend les étapes suivantes :
1) la précipitation en utilisant de la glycine à une concentration de 0,93 à 2,13 moles/l (70 à 160 g/l), et
2) la précipitation en utilisant du chlorure de sodium à une concentration de 1,17 à 2,74 moles/l (100 à 160 g/l).

2. Concentré selon la revendication 1, **caractérisé en ce qu'**il est obtenu à partir de plasma humain, d'une fraction plasmatique, de préférence, d'un cryoprécipité ou d'un matériau cellulaire modifié génétiquement.

3. Produit pharmaceutique pour traiter l'hémophilie A et le syndrome de von Willebrand, **caractérisé en ce qu'**il contient un concentré selon les revendications 1 à 2.

4. Produit pharmaceutique selon la revendication 3, **caractérisé en ce qu'**il contient des ions calcium comme stabilisateurs.

5. Produit pharmaceutique selon les revendications 3 et 4, **caractérisé en ce que** l'on effectue la précipitation (1) en utilisant de la glycine à une concentration de 0,93 à 1,46 mole/l (70 à 110 g/l).

6. Procédé de fabrication d'un concentré d'un facteur de von Willebrand contenant le facteur VIII:C, **caractérisé en ce que** l'on soumet un liquide contenant le facteur VIII:C et le facteur de von Willebrand à une précipitation fractionnée, laquelle comprend les étapes suivantes :
1) la précipitation en utilisant de la glycine à une concentration de 0,93 à 2,13 moles/l (70 à 160 g/l), et
2) la précipitation en utilisant du chlorure de sodium à une concentration de 1,17 à 2,74 moles/l (100 à 160 g/l).

7. Procédé selon la revendication 6, **caractérisé en ce que** l'on maîtrise, en réglant les concentrations de glycine et de NaCl, la quantité des multimères du facteur de von Willebrand et, par conséquent, l'activité du cofacteur ristocétine dans le concentré, dans lequel les multimères de poids moléculaire élevé précipitent, de préférence, à faibles concentrations et les multimères de faibles poids moléculaires précipitent, de préférence, à concentrations plus élevées.

8. Procédé selon l'une quelconque des revendications 6 à 7, **caractérisé en ce que** le concentré ou le produit obtenu à un stade antérieur lors de la fabrication de ce dernier, est stabilisé et pasteurisé.

9. Procédé selon l'une quelconque des revendications 6 à 8, **caractérisé en ce que** l'on mélange un cryoprécipité dissous, dans le but de permettre l'absorption du complexe prothrombinique présent en faibles quantités, à une suspension d'hydroxyde d'aluminium, puis **en ce que** l'on procède à une étape d'agitation et de séparation, **en ce que** l'on soumet le fibrinogène à une précipitation avec de la glycine et que l'on effectue une séparation, et **en ce que** l'on précipite ensuite le complexe vWF:FVIII:C, ce dernier étant soumis, après des étapes de dissolution, de stabilisation et de pasteurisation, à une précipitation fractionnée selon la revendication 6.
